# EUROPEAN PATENT SPECIFICATION

(11) **EP 4 094 671 B1**
(45) Date of publication and mention of the grant of the patent: **17.12.2025**
(21) Application number: 21765339.3
(22) Date of filing: 05.02.2021
(51) Int. Cl.: A61B 1/012, A61B 1/00, A61B 1/005, A61B 1/05, A61B 1/015, A61B 1/313, A61B 1/018

(54) **MULTIFUNCTIONAL CATHETER**
MULTIFUNKTIONSKATHETER
CATHÉTER MULTIFONCTIONNEL

(30) Priority: 04.03.2020 CN 202010143001
(43) Date of publication of application: 30.11.2022
(73) Proprietor: Micro-Tech (Nanjing) Co., Ltd., Nanjing, Jiangsu 210032 (CN)
(72) Inventor: GAO, Duangui, Nanjing, Jiangsu 210032 (CN); WANG, Jiwei, Nanjing, Jiangsu 210032 (CN); LI, Xiaochun, Nanjing, Jiangsu 210032 (CN); LIU, Fengliang, Nanjing, Jiangsu 210032 (CN); GU, Wei, Nanjing, Jiangsu 210032 (CN); GUAN, Mingxun, Nanjing, Jiangsu 210032 (CN)
(74) Representative: Jacob, Reuben Ellis
(86) International application number: PCT/CN2021/075482
(87) International publication number: WO 2021/175087

(56) References cited:
- WO-A1-2014/156149
- CN-A- 107 788 935
- CN-A- 109 805 881
- CN-U- 207 785 128
- CN-U- 209 695 154
- CN-U- 212 214 357
- US-A1- 2006 111 612
- US-A1- 2013 172 678
- US-B1- 10 433 710
- US-B1- 6 313 456

## Description

### TECHNICAL FIELD

This application relates to the field of medical instrument technology, and in particular, to a multifunctional catheter.

### BACKGROUND

In a minimally invasive operation, elongated instruments are usually used to pass through a channel of a patient body and reach a lesion location for diagnosis and treatment. Since the minimally invasive operation requires high accuracy and decidability and its visualization is particularly important, the minimally invasive operation needs an elongated endoscopic device or a catheter for auxiliary operation. The elongated catheter needs to provide a working channel for entrance and exit of the medical instrument in addition to the need to include channels for liquid entry and exit, data transmission, illumination and direction deflection, resulting that it is difficult for an outer diameter of the catheter to be small, and the diameter of the catheter is generally 5 mm or more, which is impossible to directly perform visual diagnosis and treatment operations for narrow channels such as a bile duct or a pancreatic duct.

In general, when checking a smaller channel such as a biliary tract or a pancreatic duct, a duodenum endoscope may first be inserted into the vicinity of a duodenal papilla through a digestive tract, a guiding wire is then inserted through a working channel of the endoscope, insertions of various elongated medical instruments are guided by the guiding wire, and the X-ray is used to determine the internal conditions of these channels and adjust positions of the guiding wire and instruments. The duodenal endoscope cannot enter the biliary tract and only stays outside the duodenal papilla, and this kind of operation is less intuitive, especially upon entry into branches of the biliary tract, the operation is more difficult due to the complex environment of the biliary tract.

After reaching the duodenal papilla through the working channel of the conventional duodenal endoscope, the choledochoscope continues to enter the biliary tract, which corresponds to a sub-mirror of the endoscope, and its outer diameter allows it to pass through the duodenal endoscope working channel, while having the working channel and channels for liquid (gas) to get in and out, data transmission, illumination and directional deflection, etc. The working channel of the choledochoscope itself has an inner diameter of 1.2 mm and can only allow instruments with an outer diameter of 1.1 mm or less to pass through, while outer diameter of the existing ERCP (Endoscopic Retrograde Cholangiopancreatography) medical instruments is generally 1.8-2.5 mm which are greater than 1.1 mm, so that such medical instruments cannot pass through the above choledochoscope with the working channel of an inner diameter of 1.2 mm.

US2013172678A1 describes methods and apparatuses for space-optimized visualization cathets. Some mbodiments utilize complimentary metal-oxide-semi-conductor ("CMOS") technology integrated into a CMOS camera train holder system that may be a stand-alone component for use with a visualization catheter, such as a baby endoscope, or may be fabricated/extruded as a part of the catheter itself. Some embodiments of apparatuses, methods, and equivalents thereto provide better direct visual feedback to the medical personnel performing the procedure while providing a similarly-sized outer diameter visualization catheter device having an increased space therein for additional lumens and equipment or by reducing the overall outer diameter of the visualization catheter.

### SUMMARY

The invention is defined in the appended set of claims. This application provides a multifunctional catheter to solve the problem that the existing endoscope is not suitable for the surgical environment for biliary tract.

This application provides a multifunctional catheter including: a catheter main body and a distal tube in a multi-channel structure, and the distal tube is connected with a distal end of the catheter main body; where a working channel is provided in the catheter main body and the distal end; an end face of a distal end of the distal tube is provided with a through hole communicating with the working channel; and a lens channel is further provided in the catheter main body and the distal tube, and the end face of the distal end of the distal tube is also provided with a through hole communicating with the lens channel; and
the lens channel is of a tubular-cavity structure extending along the catheter main body and the distal tube; a plurality of accommodating slots are provided on an inner wall of the lens channel to accommodate a photosensitive chip in a lens assembly through the plurality of accommodating slots.

Optionally, the plurality of accommodating slots are in a central symmetrical distribution with a central axis of the lens channel as a center.

According to the invention, the accommodating slots are right-angle V-shaped slots; and four accommodating slots are provided in the lens channel to form a rectangular space capable of accommodating the photosensitive chip in the lens assembly.

According to the invention, the rectangular space formed by the four accommodating slots is coaxial with the lens channel.

Optionally, the distal tube includes a soft end and a functional end; the soft end is located at a proximal end of the distal tube, and the functional end is located at the distal end of the distal tube.

Optionally, an end face of a through hole of the functional end communicating with the working channel is an inclined surface.

Optionally, a diameter of the working channel is greater than or equal to 48% of an outer diameter of the soft end, and the diameter of the working channel is greater than or equal to 45% of an outer diameter of the catheter main body.

Optionally, a plurality of operating channels are provided in the catheter main body and the soft end; and the functional end is provided with a plurality of connection holes communicating with the operating channels; and
a manipulating wire is provided in each of the operating channels, and a distal end of the manipulating wire is fixed in a corresponding connection hole of the functional end, so as to pull the functional end by a plurality of manipulating wires to bend the distal tube directionally.

Optionally, the catheter main body includes a reinforcement layer, and the reinforcement layer is cladded on outer walls of the catheter main body and the soft end; and the reinforcing layer is a braided mesh tubular structure including a braided metal mesh in an inner layer and a plastic matrix tube in an outer layer.

Optionally, the multifunctional catheter further includes a handle connected with the proximal end of the catheter main body, and a plurality of coaxial rotating members are provided on the handle; and
the rotating members are connected with proximal ends of the manipulating wires to pull the manipulating wires through the rotating members to control a directional bending of the distal tube.

Optionally, the handle is also provided with a working channel inlet, the working channel entrance is a 6% standard Luer taper, and the working channel inlet is communicated with the working channel.

Optionally, each rotating member is connected with two manipulating wires, and winding directions of the two manipulating wires on a rotating shaft of the rotating member are opposite, so as to produce a pulling force for controlling the directional bending of the distal tube.

Optionally, the functional end is of a cylindrical structure with an outer diameter equal to that of the soft end, and a hardness of the functional end is greater than a hardness of the soft end.

Optionally, the outer diameter of the catheter main body is less than or equal to 3.7 mm; and a diameter of the working channel is greater than or equal to 1.8 mm.

As can be seen from the above technical solutions, this application provides a multifunctional catheter including a catheter main body and a distal tube in a multi-channel structure. Where a working channel and a lens channel are provided in the catheter main body and the distal tube, and an end face of a distal end of the distal tube is provided with a through hole communicating with the lens channel; the lens channel is of a tubular-cavity structure extending along the catheter main body and the soft end; and a plurality of accommodating slots are provided on an inner wall of the lens channel, so as to accommodate a photosensitive chip in a lens assembly through the plurality of accommodating slots. The catheter provided by this application may form a space for accommodating the photosensitive chip through the plurality of accommodating slots in the lens channel, so that the lens assembly can be accommodated without increasing a diameter of the lens channel, and the overall diameter of the catheter is reduced so as to adapt to the environment of a biliary tract that is more complex relative to a digestive tract.

### BRIEF DESCRIPTION OF DRAWINGS

FIG. 1 is a structural schematic view of a multifunctional catheter of this application;
FIG. 2 is a cross-sectional structural schematic view of a catheter main body of this application;
FIG. 3 is a cross-sectional structural schematic view of a soft end of this application;
FIG. 4 is a three-dimensional structural schematic view of a handle of this application;
FIG. 5 is a cross-sectional structural schematic view of a functional end of this application;
FIG. 6 is a three-dimensional structural schematic view of the functional end of this application; and
FIG. 7 is a cross-sectional structural schematic view of another functional end of this application.

### DESCRIPTION OF EMBODIMENTS

Embodiments will be described in detail below, examples of which are illustrated in the drawings. When the following description refers to the drawings, the same numbers in different drawings refer to the same or similar elements unless otherwise indicated. The implementations described in the following embodiments do not represent all implementations consistent with this application, and are merely examples of systems and methods consistent with some aspects of this application as recited in the claims.

In the technical solutions provided by this application, for ease of description, an end of the entire catheter that is placed into the human body is referred as a distal end, and this end is mainly used to implement operation action on tissues; and an end of the entire catheter that is located outside the human body is referred as a proximal end, which is mainly used for operations of the surgical operator. Unless otherwise specified in this application, the distal end of each component refers to an end close to an interior side of the human body, and the proximal end of each component refers to an end close to an exterior side of the human body.

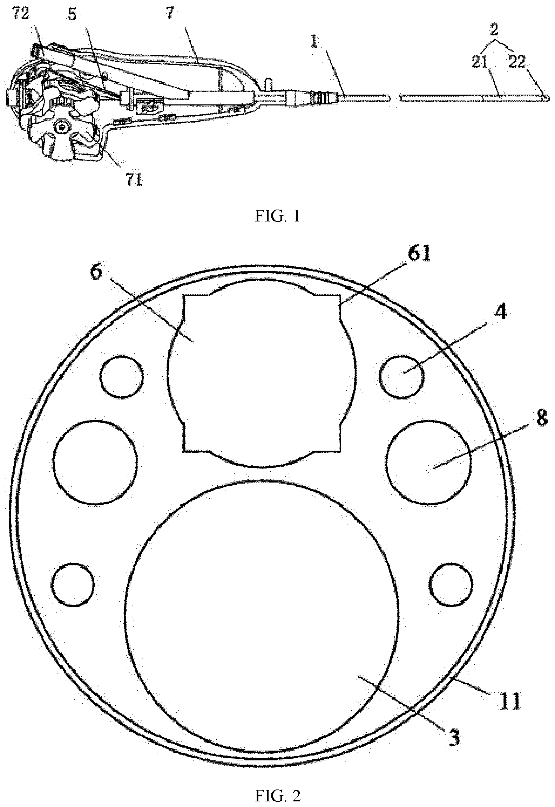

Referring to FIG. 1, it is a structural schematic view of a multifunctional catheter of this application. As can be seen from FIG. 1 and FIG. 2, a multifunctional catheter provided by this application includes a catheter main body 1 and a distal tube 2. The catheter main body 1 and the distal tube 2 are each in multi-channel structures. The distal tube 2 is connected to a distal end of the catheter main body 1 as an extension of the distal end of the catheter main body 1. In practical application, a length of the catheter main body 1 should be sufficient to satisfy extending from the outside of the patient's body to a lesion location of a channel inside the patient's body, and a length of the distal tube 2 should be capable of satisfying travel requirements of the maximum turning angle of the distal end of the catheter.

A working channel 3 is provided in the catheter main body 1 and the distal tube 2; a through hole communicating with the working channel 3 is provided on a distal end face of the distal catheter 2; a lens channel 6 is provided in the catheter main body 1 and the distal end tube 2, and a through hole communicating with the lens channel 6 is provided on the distal end face of the distal catheter 2.

In some embodiments, the distal tube 2 includes a soft end 21 at its proximal end and a functional end 22 at its distal end. Where the functional end 22 may be configured to be installed with various endoscope detection components, such as a camera, a light source, etc., and is also configured to connect a manipulating wire that drives the turning of the distal tube 2. The soft end 21 is configured to connect the catheter main body 1 and the functional end 22. In this embodiment, a hardness of the soft end 21 is smaller than that of the catheter main body 1, so that the soft end 21 is more easily deformed relative to the catheter main body 1, so as to be capable of smoothly enter the channels in the patient's body under the guidance of a guiding wire, and the resistance of the channel tissue wall on the catheter can also be reduced, thereby avoiding the catheter from scratching the tissue in the patient's body.

It is to be noted that in this application, the hardness of the soft end 21 being smaller than that of the catheter main body 1 means that the soft end 21 is more easily to be deformed relative to the catheter main body 1, rather than simply the difference in hardness of the material itself. Therefore, in practical application, the hardness of the soft end 21 being smaller than that of the catheter main body 1 may be realized by different materials, and may also be realized by an easily deformable structure. When the difference in hardness is realized by different materials, the catheter main body 1 may be made of a hard material, for example, a plastic material such as polytetrafluoroethylene (PTFE), polyamide (PA) and polyether block amide (PEBAX); the soft end 21 may be made of a soft material, such as a polytetrafluoroethylene (PTFE), polyamide (PA), silicone rubber, polyurethane (PU) and polyether block amide (PEBAX) having a lower hardness of quenching and tempering. When the difference in hardness is realized by the easily deformable structure, the soft end 21 may be provided as a corrugated structure, a thin tube structure, a multi-channel structure or the like that is easily deformed.

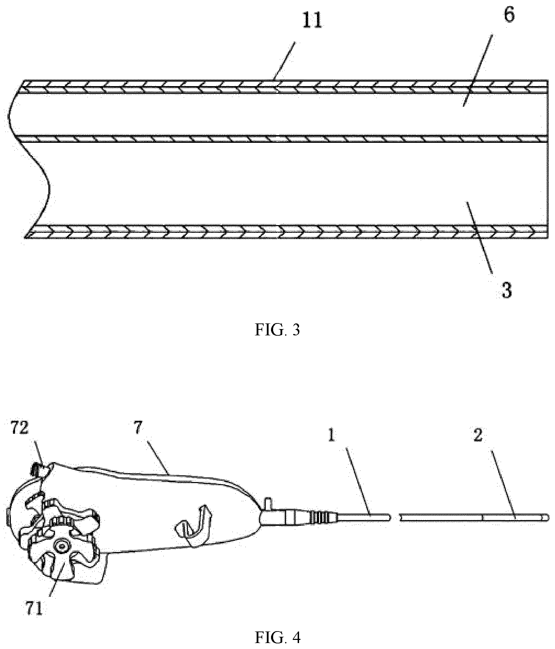

In the technical solution provided by this application, as shown in FIG. 3, the working channel 3 is provided in the catheter main body 1 and the soft end 21; the through hole communicating with the working channel 3 is provided on the functional end 22. The working channel 3 may be configured to insert auxiliary instruments for further diagnosis and treatment in practical application, such as biopsy forceps and laser lithotripsy instruments, etc., and may also be configured to deliver medicines through the working channel 3, and to flush the channel and transport fluids in the channel outside the body. In order to facilitate the operation of auxiliary instruments, a diameter of the working channel 3 is greater than or equal to 48% of an outer diameter of the soft end 21, and the diameter of the working channel 3 is greater than or equal to 45% of an outer diameter of the catheter main body 1.

In practical application, the greater the diameter of the working channel 3, the larger a space for surgical operations, which is more beneficial to conducting the surgical operations. However, due to the limitation of the channel inside the human body, the outer diameters of the catheter main body 1 and the soft end 21 cannot be very large, so the diameter of the working channel 3 cannot be set to be very large. Taking a bile duct and a pancreatic duct as an example, due to the influence of inner diameters of channels of the bile duct and pancreatic duct, the outer diameter of the catheter main body 1 is less than or equal to 3.7 mm, that is, the diameter of the working channel 3 should be designed with reference to this parameter limit.

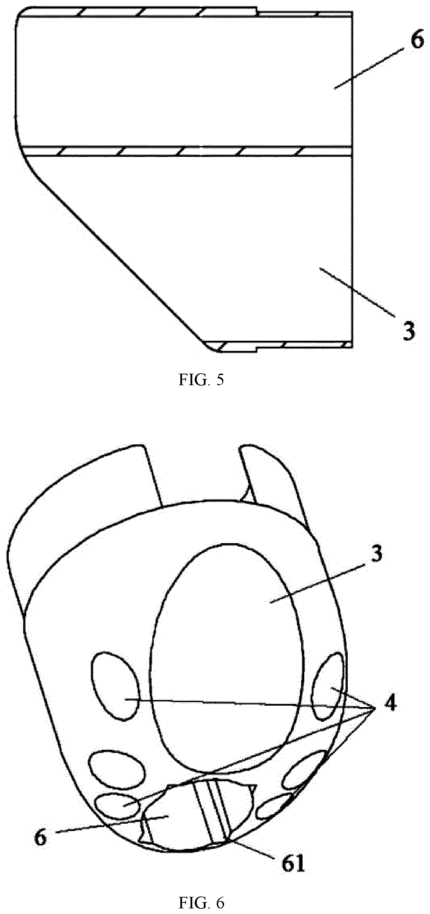

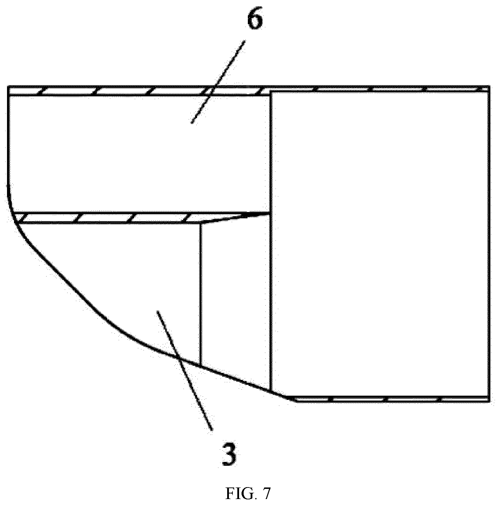

In some embodiments, an end face of the through hole of the functional end 22 communicating with the working channel 3 is an inclined surface, and such inclined surface may reduce the resistance of the catheter when traveling in a digestive tract, so as to reach a position of illness. Exemplarily, as shown in FIG. 5 and FIG. 6, an inclination angle of the inclined surface may be 45°. Obviously, the end face of the through hole communicating with the working channel 3 may also be formed by splicing a plurality of surfaces with different inclination angles. As shown in FIG. 7, the end face of the functional end 22, when transitioning from a position of the lens channel 6 to the working channel 3, may be composed of two inclined surfaces, so that a shape of the end face gradually tends to be sharp from being gentle, which is beneficial to entering the digestive tract.

In the technical solutions provided by this application, as shown in FIG. 2, FIG. 5 and FIG. 6, the lens channel 6 is further provided in the catheter main body 1 and the soft end 21, and the functional end 22 is provided with a through hole communicating with the lens channel 6. A lens assembly may be provided at the end face of the through hole on the functional end 22 to capture images of the environment in the digestive tract, and wire components such as data cables of the lens assembly may be disposed in the lens channel 6 so as to transmit data of the images captured by the lens assembly to a display device for display.

The lens assembly generally includes a lens and a photosensitive chip. The image in the digestive tract reaches the photosensitive chip through the refraction of the lens to generate image signal. A volume of the lens assembly may be limited by a shape of the photosensitive chip, that is, the photosensitive chip may not be too small so as to avoid too low resolution of the captured image. And the lens in the lens assembly may be in various shapes, but the photosensitive chip may only be made into a rectangular structure. During assembling, the photosensitive chip in the rectangular structure needs to satisfy the fact that an inner diameter of the lens channel 6 is greater than a diagonal length of the photosensitive chip, resulting in a larger accommodating space required for the lens assembly.

For example, for a square photosensitive chip of 1.12 mm×1.12 mm, its diagonal length is 1.697 mm, so the lens channel 6 needs to have an inner diameter of more than 1.7 mm in order to accommodate the photosensitive chip; and for a rectangular photosensitive chip, the lens channel 6 needs to have a greater inner diameter. When the inner diameter of the lens channel 6 is relative large, the lens channel 6 may occupy the entire space of the catheter, so that when the overall outer diameter of the catheter is limited, the available space of the working channel 3 is compressed, and the inner diameter of the working channel 3 is also limited to be a lower value.

For this reason, in this application, the lens channel 6 is a circular tubular-cavity structure extending along the catheter main body 1 and the soft end 21. A plurality of accommodating slots 61 are provided on an inner wall of the lens channel 6 so as to accommodate the photosensitive chip in the lens assembly through the plurality of accommodating slots 61. In practical application, the plurality of accommodating slots 61 form a rectangular space for accommodating the photosensitive chip, so as to adapt to the shape of the photosensitive chip and reduce the diameter of the circular tubular-cavity of the lens channel 6.

In order to form the rectangular structure, the accommodating slot 61 is a right-angled V-shaped slot. Four accommodating slots 61 are provided in lens channel 6 to form a rectangular space capable of accommodating the photosensitive chip in the lens assembly. For example, the four accommodating slots 61 are right-angle V-shaped slots, forming a rectangular accommodating space of 1.12 mm×1.12 mm, which may accommodate a photosensitive chip with a maximum size of 1.12 mm×1.12 mm. At this case, the diameter of the circular tubular-cavity of the lens channel 6 is 1.36 mm or less. However, if the above accommodating slots 61 are not used, a circular tubular-cavity with a diameter above 1.7 mm is required to accommodate the photosensitive chip of 1.12×1.12 mm. It can be seen that the space for accommodating the photosensitive chip is formed by the plurality of accommodating slots 61 in the lens channel 6, and the lens assembly may be installed without increasing the diameter of the lens channel 6, thereby reducing the overall diameter of the catheter.

Since the diameter of the lens channel 6 may be smaller and then the space occupied by the lens channel 6 as a whole relative to the catheter is reduced, on the premise of the limited outer diameter of the catheter, a larger working channel 3 may be obtained. For example, on the premise that the outer diameter of the catheter main body is less than or equal to 3.7 mm, the diameter of the working channel may be greater than or equal to 1.8 mm, so as to facilitate the passage of conventional ERCP medical instruments.

In addition, comparing with the traditional cylindrical tubular-cavity structure, the rectangular structure formed by the four accommodating slots 61 also facilitates fixing the lens assembly, that is, the photosensitive chip is confined by the rectangular sides of the accommodating slots 61, thereby preventing the lens component from rotating in the lens channel 6 during operation, and facilitating to maintain the stability of the image picture during the surgical operation.

The rectangular space formed by the four accommodating slots 61 is coaxial with the lens channel 6, which may realize that on the basis of accommodating the photosensitive chip of the lens assembly, a central position of the photosensitive chip can be limited to a central position of the lens channel 6, thereby being convenient for installation and fixation. At the same time, the rectangular space formed by the accommodating slots 61 is coaxial with the lens channel 6, which also facilitates establishing a connection relation between the photosensitive chip and the optical fiber data in the lens assembly, thereby reducing redundant wiring and circuit board layout and saving the space in the lens channel 6.

In some embodiments, the plurality of accommodating slots 61 are in a central symmetrical distribution with a central axis of the lens channel 6 as a center; among the plurality of accommodating slots 61, a distance between two accommodating slots 61 with a diagonal relationship is greater than or equal to the diagonal length of the photosensitive chip.

For example, in a cross-section shape of the catheter shown in FIG. 2, the lens channel 6 includes four accommodating slots 61, which are respectively located at an upper left corner, an upper right corner, a lower left corner and a lower right corner of the lens channel 6 and in a central symmetrical distribution with the central axis of the lens channel 6 as a center. In addition, in order to accommodate the photosensitive chip, among the four accommodating slots 61, a distance between the two accommodating slots 61 located at the upper left corner and the lower right corner of the lens channel 6 should be greater than or equal to the diagonal length of the photosensitive chip. Similarly, a distance between the two accommodating slots 61 located at the upper right corner and the lower left corner of the lens channel 6 is also greater than or equal to the diagonal length of the photosensitive chip, so that the photosensitive chip can be accommodated, and sides of the photosensitive chip are limited to prevent it from rotating.

In this embodiment, the lens assembly includes the photosensitive chip and a data cable, where the photosensitive chip may acquire images of the channel in real time, and transmit them to the display device located at the proximal end of the catheter through the data cable, so as to show the travelling of the catheter in the channel and the movement of surgical instruments in actual surgical operations.

In this application, the guiding wire in the endoscope may be guided to control the distal end of the catheter to realize a turning action. However, when the distal end of the catheter runs into the bile duct that cannot be reached by the endoscope, a manipulating wire 5 is needed for controlling the turning of the distal end of the catheter. That is, in some embodiments provided in this application, a plurality of operating channels 4 are further provided in the catheter main body 1 and the soft end 21; a plurality of connection holes communicating with the operating channels 4 are provided on the functional end 22; the manipulating wire 5 is provided in each of the operating channels 4; and a distal end of the manipulating wire 5 is fixed in the corresponding connection hole of the functional end 2 so as to pull the functional end 22 by the plurality of manipulating wires 5 to bend the distal tube 2 directionally.

In practical application, one end of the manipulating wire 5 is connected with the functional end 22, and the other end of the manipulating wire 5 extends from the distal end of the catheter to its proximal end, so the manipulating wire 5 may be pulled at the proximal end of the catheter so as to pull the functional end 22 to develop a movement tendency, thereby forming a serpentine bending on the distal tube 2. Obviously, the number of the manipulating wires 5 that can satisfy the surgical operation should be at least two, and in order to realize a more flexible turning operation, the number of the manipulating wires 5 and the number of the operation channels 4 may be appropriately increased.

In this embodiment, in order to satisfy requirements for fixing the distal end of the manipulating wire 5, the functional end 22 may be provided as a metal or plastic structure, and is connected with the manipulating wire 5 by welding or insert injection molding, so that the distal end of the manipulating wire 5 is fixed and connected to the functional end 22, realizing that the turning of the distal tube 2 can be controlled by adjusting the length of the manipulating wire 5. A diameter of the manipulating wire 5 should be below 0.35 mm, and a firm and wear-resistant metal wire or plastic fiber may be selected.

As can be seen from the above technical solutions, as shown in FIG. 6, in order to realize an interconnected channel body, the catheter main body 1 in this application is consistent with the soft end 21 and the functional end 22 in the arrangement manner of the channels. For example, the catheter main body 1 is provided with one working channel 3, four operating channels 4 and one lens channel 6. That is, a deflection function in four different directions may be satisfied, which is not only simple in process, but also saves a lot of space. At the same time, it may realize functions of turning, working channel, liquid injection and suction, optical vision, etc. The outer diameter of the catheter main body 1 is reduced to 3.7 mm or less, which may satisfy the use of most ERCP instruments, that is, it may satisfy the visual diagnosis and treatment requirements of the bile duct and the pancreatic duct.

In some embodiments of this application, the catheter main body 1 includes a reinforcement layer 11, and the reinforcement layer 11 is cladded on outer walls of the catheter main body 1, the soft end 21 and the functional end 22. On the one hand, the reinforcing layer 11 may enhance the rigidity of the whole catheter, so as to facilitate the delivery of the catheter to the channel in the patient's body; on the other hand, the catheter main body 1, the soft end 21 and the functional end 22 are communicated through the reinforcement layer 11, so that when the catheter main body 1 and the distal tube 2 are not made of the same material or not in an integral structure, they may still be connected as a whole, thereby avoiding the deformation of the channel and the catheter caused by the use of a connector.

In some embodiments, the reinforcing layer 11 is a braided mesh-tubular structure, including a braided metal mesh at an inner layer and a plastic matrix tube at an outer layer. A thickness of the tube wall of the reinforcing layer 11 should be at least 0.1 mm to maintain sufficient connection strength. Therefore, when the outer diameter of the catheter main body 1 is less than or equal to 3.7 mm, the actual internal diameter thereof should be less than 3.5 mm. With the current extrusion technology and material characteristics, the effective limit thickness of the walls of the catheter main body 1 and the soft end 21 (for example, the soft end 21 is made of PEBAX, silica gel, etc.) is 0.1 mm, so the diameter of the internal space for the multi-channel design is 3.3 mm or less, and the diameter of the working channel may be designed to be 1.8 mm or more. That is, the diameter of the working channel 3 is greater than or equal to 1.8 mm.

In some embodiments of this application, as shown in FIG. 4, the multifunctional catheter also includes a handle 7 that is connected with the proximal end of the catheter main body 1, and the handle 7 is also provided with a plurality of coaxial rotating members 71. The rotating members 71 are connected with the proximal ends of the manipulating wires 5, so that the manipulating wires 5 are drawn by the rotating members 71 to control the directional bending of the distal tube 2. Where each of the rotating members 71 is connected with two manipulating wires 5, and winding directions of the two manipulating wires 5 on a rotating shaft of each rotating member 71 are opposite, so as to produce a pulling force for controlling the directional bending of the distal tube 2. In some embodiments, the handle 7 is also provided with a working channel inlet 72, the working channel inlet 72 is a 6% standard Luer taper, and the working channel inlet 72 is communicated with the working channel 3.

In this embodiment, the proximal end of the catheter main body 1 includes the handle 7, and the handle 7 includes a pair of coaxial rotating members 71, i.e., rotating wheels, and each of the rotating wheels is connected with one manipulating wire 5 at each of both sides of its rotating shaft. When the multifunctional catheter is used alone or in conjunction with a duodenoscope to reach a target channel, a doctor holds the handle 7 with one hand or hangs the handle 7 near a working channel of other endoscopes (such as a duodenoscope) used cooperatively with the multifunctional catheter.

By means of the twisting of the coaxial rotating members 71 on the handle 7, the rotating shafts of the two coaxial rotating wheels in the handle 7 are driven to rotate respectively. When one of the rotating wheels rotates clockwise, the two symmetrical manipulating wires 5 to which it is connected will produce a relative movement in the operating channel 4 in the catheter main body 1 and the distal tube 2, and respectively generate a pulling force and a pushing force in opposite directions, so that the functional end 22 connected with the manipulating wires 5 is inclined to compress the soft end 21 in the distal tube 2, and the distal tube 2 is bent in a predetermined direction. When the rotating wheel is rotated counterclockwise, the distal tube 2 will bend in another direction. Similarly, when the other rotating wheel rotates clockwise and counterclockwise, the distal tube 2 is also driven to bend in other directions.

In this embodiment, the doctor controls a direction that the distal end tube 2 enters in the patient's body and the channel of the cooperative endoscope, and corresponding observation direction by rotating the rotating members 71 in the handle 7, and simultaneously may control a depth of the distal tube 2 entering the channel by pushing the handle 7 at the proximal end of the catheter main body 1. In addition, the working channel inlet 72 with a structure of a 6% standard Luer taper is provided at the proximal end of the handle 7, and is connected with the working channel 3. A doctor may insert auxiliary instruments, such as biopsy forceps and laser lithotripsy instruments, through the working channel inlet 72 for further diagnosis and treatment, and may also inject liquid through the working channel inlet 72 to flush the channel and wash the fluid in the channel out of the body.

In some embodiments provided by this application, the functional end 22 is a cylindrical structure, and its outer diameter is the same as that of the soft end 21, and the hardness of the functional end 22 is greater than the hardness of the soft end 21. In practical application, the functional end 22 may be in a cylindrical structure made of hard plastic or stainless steel, and the cylindrical structure is provided with through holes corresponding to the multiple channels of the soft end 21. The functional end 22 may be pre-drilled with a plurality of installation holes, such as a camera hole, a hole for fixing the manipulating wire 5, and a drug hole or a liquid injection hole that is communicated with a liquid injection channel 8, through hard materials. Therefore, the hardness of the functional end 22 is greater than the hardness of the soft end 21, which may facilitate the fixing of various installation components and make the overall working process more stable. In addition, the selection of the functional end 22 with higher hardness is also convenient for processing into holes and improves the process accuracy.

As can be seen from above technical solutions, the multifunctional catheter provided in above embodiments includes the catheter main body 1 and the distal tube 2 in a multi-channel structure. Where the distal tube 2 includes the soft end 21 at a proximal end and the functional end 22 at a distal end; a hardness of the soft end 21 is less than a hardness of the catheter main body 1; the working channel is provided in the catheter main body 1 and the soft end 21; the functional end 22 is provided with a through hole communicating with the working channel 3; a diameter of the working channel 3 is greater than or equal to 51% of an outer diameter of the soft end 21, and the diameter of the working channel 3 is greater than or equal to 49% of an outer diameter of the catheter main body 1. The catheter provided in this application may realize a turning function by the cooperation between the soft end 21 of the distal tube 2 and the working channel 3, so that the distal end of the catheter may adapt to the environment of the biliary tract that is more complex relative to the digestive tract.

Meanwhile, the lens channel 6 has a circular tubular-cavity structure extending along the catheter main body 1 and the soft end 21; a plurality of accommodating slots 61 are provided on an inner wall of the lens channel 6 to accommodate the photosensitive chip in the lens assembly by the accommodating slots 61. The catheter provided by this application may form a space for accommodating the photosensitive chip by the plurality of accommodating slots 61 in the lens channel 6, so that the lens assembly can be accommodated without increasing the diameter of the lens channel 6, and the overall diameter of the catheter is reduced so as to adapt to the environment of the biliary tract that is more complex relative to the digestive tract.

In addition, regarding the multifunctional catheter provided by this application, a conventional instrument with a diameter of 1.8 mm can pass through the working channel of the catheter, which is convenient for the doctor to use, saves the manufacturing cost, reduces the price of the instruments that are cooperatively used, and further reduces the burden of patient.

Similar parts between the embodiments provided in this application may be referred to each other. The specific implementations provided above are just a few examples under the general concept of this application, and do not constitute a limitation on the protection scope of this application. For those skilled in the art, any other implementations expanded according to the solutions of this application without creative work fall within the protection scope of this application.

## Claims

1. A multifunctional catheter, comprising:
a catheter main body (1) having a multi-channel structure;
a distal tube (2) having a multi-channel structure and connected with a distal end of the catheter main body (1);
wherein a working channel (3) is provided in the catheter main body (1) and the distal tube (2); an end face of a distal end of the distal tube (2) is provided with a through hole communicating with the working channel (3); and a lens channel (6) is provided in the catheter main body (1) and the distal tube (2), and the end face of the distal end of the distal tube (2) is provided with a through hole communicating with the lens channel (6);
the lens channel (6) is of a tubular-cavity structure extending along the catheter main body (1) and the distal tube (2); a plurality of accommodating slots (61) are provided on an inner wall of the lens channel (6) to accommodate a photosensitive chip in a lens assembly through the plurality of accommodating slots (61);
the accommodating slots (61) are right-angle V-shaped slots; and four accommodating slots (61) are provided in the lens channel (6) to form a rectangular space capable of accommodating the photosensitive chip in the lens assembly;
**characterized in that**
the rectangular space formed by the four accommodating slots (61) is coaxial with the lens channel (6).

2. The multifunctional catheter according to claim 1, **characterized in that** the four accommodating slots (61) are in a central symmetrical distribution with a central axis of the lens channel (6) as a center; among the four accommodating slots (61), a distance between two accommodating slots (61) having a diagonal relationship is greater than or equal to a diagonal length of the photosensitive chip.

3. The multifunctional catheter according to claim 1, **characterized in that** the distal tube (2) comprises a soft end (21) and a functional end (22); the soft end (21) is located at a proximal end of the distal tube (2), and the functional end (22) is located at the distal end of the distal tube (2).

4. The multifunctional catheter according to claim 3, **characterized in that** an end face of a through hole of the functional end (22) communicating with the working channel (3) is an inclined surface.

5. The multifunctional catheter according to claim 3, **characterized in that** a diameter of the working channel (3) is greater than or equal to 48% of an outer diameter of the soft end (21), and the diameter of the working channel (3) is greater than or equal to 45% of an outer diameter of the catheter main body (1).

6. The multifunctional catheter according to claim 3, **characterized in that** a plurality of operating channels (4) are further provided in the catheter main body (1) and the soft end (21); and the functional end (22) is provided with a plurality of connection holes communicating with the operating channels (4); and
a manipulating wire (5) is provided in each of the operating channels (4), and a distal end of the manipulating wire (5) is fixed in a corresponding connection hole of the functional end (22), so as to pull the functional end (22) by a plurality of manipulating wires (5) to bend the distal tube (2) directionally.

7. The multifunctional catheter according to claim 3, **characterized in that** the catheter main body (1) comprises a reinforcement layer (11), and the reinforcement layer (11) is cladded on outer walls of the catheter main body (1) and the soft end (21); and the reinforcing layer (11) is a braided mesh-tubular structure, comprising a braided metal mesh in an inner layer and a plastic matrix tube in an outer layer.

8. The multifunctional catheter according to claim 3, **characterized in that** the functional end (22) is of a cylindrical structure with an outer diameter equal to that of the soft end (21), and the functional end (22) has a hardness greater than a hardness of the soft end (21).

## Patentansprüche

1. Multifunktionskatheter, umfassend:
einen Katheterhauptkörper (1), der eine Mehrkanalstruktur aufweist;
eine distale Röhre (2), die eine Mehrkanalstruktur aufweist und mit einem distalen Ende des Katheterhauptkörpers (1) verbunden ist;
wobei in dem Katheterhauptkörper (1) und der distalen Röhre (2) ein Arbeitskanal (3) bereitgestellt ist; eine Endfläche eines distalen Endes der distalen Röhre (2) mit einem Durchgangsloch versehen ist, das mit dem Arbeitskanal (3) in Verbindung steht; und in dem Katheterhauptkörper (1) und der distalen Röhre (2) ein Linsenkanal (6) bereitgestellt ist und die Endfläche des distalen Endes der distalen Röhre (2) mit einem Durchgangsloch versehen ist, das mit dem Linsenkanal (6) in Verbindung steht;
wobei der Linsenkanal (6) eine sich entlang des Katheterhauptkörpers (1) und der distalen Röhre (2) erstreckende Röhrenhohlraumstruktur aufweist; an einer inneren Wand des Linsenkanals (6) eine Vielzahl von Aufnahmenuten (61) bereitgestellt ist, um einen photosensitiven Chip in einer Linsenanordnung über die Vielzahl von Aufnahmenuten (61) aufzunehmen;
wobei die Aufnahmenuten (61) rechtwinklig Y-förmige Nuten sind; und in dem Linsenkanal (6) vier Aufnahmenuten (61) bereitgestellt sind, um einen rechteckigen Raum zu bilden, der in der Lage ist, den photosensitiven Chip in der Linsenanordnung aufzunehmen;
**dadurch gekennzeichnet, dass**
der von den vier Aufnahmenuten (61) gebildete rechteckige Raum zu dem Linsenkanal koaxial ist.

2. Multifunktionskatheter nach Anspruch 1, **dadurch gekennzeichnet, dass** die vier Aufnahmenuten (61) in einer mittigen symmetrischen Verteilung mit einer Mittelachse des Linsenkanals (6) als Mitte vorliegen; unter den vier Aufnahmenuten (61) ein Abstand zwischen zwei Aufnahmenuten (61) mit einer diagonalen Beziehung größer als oder gleich einer Diagonalenlänge des photosensitiven Chips ist.

3. Multifunktionskatheter nach Anspruch 1, **dadurch gekennzeichnet, dass** die distale Röhre (2) ein weiches Ende (21) und ein Funktionsende (22) umfasst; sich das weiche Ende (21) an einem proximalen Ende der distalen Röhre (2) befindet und sich das Funktionsende (22) an dem distalen Ende der distalen Röhre (2) befindet.

4. Multifunktionskatheter nach Anspruch 3, **dadurch gekennzeichnet, dass** eine mit dem Arbeitskanal (3) in Verbindung stehende Endfläche eines Durchgangslochs des Funktionsendes (22) eine schräge Fläche ist.

5. Multifunktionskatheter nach Anspruch 3, **dadurch gekennzeichnet, dass** ein Durchmesser des Arbeitskanals (3) größer als oder gleich 48 % eines Außendurchmessers des weichen Endes (21) ist und der Durchmesser des Arbeitskanals (3) größer als oder gleich 45 % eines Außendurchmessers des Katheterhauptkörpers (1) ist.

6. Multifunktionskatheter nach Anspruch 3, **dadurch gekennzeichnet, dass** in dem Katheterhauptkörper (1) und dem weichen Ende (21) ferner eine Vielzahl von Operationskanälen (4) bereitgestellt ist; und das Funktionsende (22) mit einer Vielzahl von Verbindungslöchern versehen ist, die mit den Operationskanälen (4) in Verbindung stehen; und
in jedem der Operationskanäle (4) ein Manipulierdraht (5) bereitgestellt ist und ein distales Ende des Manipulierdrahts (5) in einem entsprechenden Verbindungsloch des Funktionsendes (22) befestigt ist, um das Funktionsende (22) durch eine Vielzahl von Manipulierdrähten (5) zu ziehen, um die distale Röhre (2) direktional zu biegen.

7. Multifunktionskatheter nach Anspruch 3, **dadurch gekennzeichnet, dass** der Katheterhauptkörper (1) eine Verstärkungsschicht (11) umfasst und die Verstärkungsschicht (11) auf äußere Wände des Katheterhauptkörpers (1) und des weichen Endes (21) aufgetragen ist; und die Verstärkungsschicht (11) eine geflochtene Netzröhrenstruktur ist, die ein geflochtenes Metallnetz in einer inneren Schicht und eine Kunststoffmatrixröhre in einer äußeren Schicht umfasst.

8. Multifunktionskatheter nach Anspruch 3, **dadurch gekennzeichnet, dass** das Funktionsende (22) eine zylindrische Struktur mit einem Außendurchmesser gleich demjenigen des weichen Endes (21) aufweist und das Funktionsende (22) eine Härte aufweist, die größer als eine Härte des weichen Endes (21) ist.

## Revendications

1. Cathéter multifonctionnel comprenant :
un corps principal de cathéter (1) ayant une structure à canaux multiples ;
un tube distal (2) ayant une structure à canaux multiples et connecté avec une extrémité distale du corps principal de cathéter (1) ;
dans lequel un canal de travail (3) est fourni dans le corps principal de cathéter (1) et le tube distal (2) ; une face d'extrémité d'une extrémité distale du tube distal (2) est pourvue d'un trou traversant communiquant avec le canal de travail (3) ; et un canal de lentille (6) est fourni dans le corps principal de cathéter (1) et le tube distal (2), et la face d'extrémité de l'extrémité distale du tube distal (2) est pourvue d'un trou traversant communiquant avec le canal de lentille (6) ;
le canal de lentille (6) est d'une structure à cavité tubulaire s'étendant le long du corps principal de cathéter (1) et du tube distal (2) ; une pluralité de fentes de réception (61) sont fournies sur une paroi interne du canal de lentille (6) pour recevoir une puce photosensible dans un ensemble de lentille à travers la pluralité de fentes de réception (61) ;
les fentes de réception (61) sont des fentes en forme de V à angle droit ; et quatre fentes de réception (61) sont fournies dans le canal de lentille (6) pour former un espace rectangulaire capable de recevoir la puce photosensible dans l'ensemble de lentille ;
**caractérisé en ce que**
l'espace rectangulaire formé par les quatre fentes de réception (61) est coaxial avec le canal de lentille (6).

2. Cathéter multifonctionnel selon la revendication 1, **caractérisé en ce que** les quatre fentes de réception (61) sont dans une distribution symétrique centrale avec un axe central du canal de lentille (6) comme centre ; entre les quatre fentes de réception (61), une distance entre deux fentes de réception (61) ayant une relation diagonale est supérieure ou égale à une longueur diagonale de la puce photosensible.

3. Cathéter multifonctionnel selon la revendication 1, **caractérisé en ce que** le tube distal (2) comprend une extrémité souple (21) et une extrémité fonctionnelle (22) ; l'extrémité souple (21) est située au niveau d'une extrémité proximale du tube distal (2), et l'extrémité fonctionnelle (22) est située au niveau de l'extrémité distale du tube distal (2).

4. Cathéter multifonctionnel selon la revendication 3, **caractérisé en ce qu'**une face d'extrémité d'un trou traversant de l'extrémité fonctionnelle (22) communiquant avec le canal de travail (3) est une surface inclinée.

5. Cathéter multifonctionnel selon la revendication 3, **caractérisé en ce qu'**un diamètre du canal de travail (3) est supérieur ou égal à 48 % d'un diamètre externe de l'extrémité souple (21), et le diamètre du canal de travail (3) est supérieur ou égal à 45 % d'un diamètre externe du corps principal de cathéter (1).

6. Cathéter multifonctionnel selon la revendication 3, **caractérisé en ce qu'**une pluralité de canaux de fonctionnement (4) sont en outre fournis dans le corps principal de cathéter (1) et l'extrémité souple (21) ; et l'extrémité fonctionnelle (22) est pourvue d'une pluralité de trous de connexion communiquant avec les canaux de fonctionnement (4) ; et
un fil de manipulation (5) est fourni dans chacun des canaux de fonctionnement (4), et une extrémité distale du fil de manipulation (5) est fixée dans un trou de connexion correspondant de l'extrémité fonctionnelle (22), de manière à tirer l'extrémité fonctionnelle (22) par une pluralité de fils de manipulation (5) pour plier le tube distal (2) de manière directionnelle.

7. Cathéter multifonctionnel selon la revendication 3, **caractérisé en ce que** le corps principal de cathéter (1) comprend une couche de renforcement (11), et la couche de renforcement (11) est plaquée sur des parois externes du corps principal de cathéter (1) et de l'extrémité souple (21) ; et la couche de renforcement (11) est une structure tubulaire à maille tressée, comprenant une maille métallique tressée dans une couche interne et un tube à matrice en plastique dans une couche externe.

8. Cathéter multifonctionnel selon la revendication 3, **caractérisé en ce que** l'extrémité fonctionnelle (22) est d'une structure cylindrique avec un diamètre externe égal à celui de l'extrémité souple (21), et l'extrémité fonctionnelle (22) a une dureté supérieure à une dureté de l'extrémité souple (21).
